Europäisches Patentamt

European Patent Office

Office européen des brevets

(19)

(11) Publication number: **0 109 993**
**B1**

(12) **EUROPEAN PATENT SPECIFICATION**

(45) Date of publication of patent specification: **08.02.89**

(51) Int. Cl.⁴: **A 61 K 35/64, A 61 K 7/48**

(21) Application number: **82306395.3**

(22) Date of filing: **01.12.82**

(54) **Method for extracting propolis and water soluble dry propolis powder obtained thereby and cosmetic and pharmaceutical preparations containing same.**

(43) Date of publication of application:
**13.06.84 Bulletin 84/24**

(45) Publication of the grant of the patent:
**08.02.89 Bulletin 89/06**

(84) Designated Contracting States:
**AT BE CH DE FR GB IT LI LU NL SE**

(56) References cited:
**DE-B-1 037 651**
**US-A-3 960 329**

**BIOLOGICAL ABSTRACTS, vol. 68, 1979, abstract no. 72464, Philadelphia, Pennsylvania, USA**

(73) Proprietor: **Sosnowski, Zenon M.**
**1081 Beauty Avenue**
**Winnipeg Manitoba R2P 0E9 (CA)**

(72) Inventor: **Sosnowski, Zenon M.**
**1081 Beauty Avenue**
**Winnipeg Manitoba R2P 0E9 (CA)**

(74) Representative: **Lambert, Hugh Richmond et al**
**D. YOUNG & CO. 10 Staple Inn**
**London, WC1V 7RD (GB)**

**The file contains technical information submitted after the application was filed and not included in this specification**

EP 0 109 993 B1

Courier Press, Leamington Spa, England.

## Description

Propolis is a resinous compound collected by honey bees from various plants and the buds of different trees. It is also known as "honey bee glue" and is used by the bees to coat parts of the interior of the hive and to seal the cracks and crevices of the hive.

The present invention is concerned with the extraction of propolis from clean raw propolis as well as unprocessed beeswax to obtain a dry propolis powder, with the water soluble dry propolis powder thereby obtainable, and with pharmaceutical, medicinal and cosmetic preparations containing same.

The existence of propolis has of course long been recognized by apiarists. In fact, it has been variously described as both a blessing and a curse. While the substance has been largely ignored as a necessary sticky evil associated with beekeeping, some rather surprising studies of propolis have been made with particular regard to commercial and the therapeutic uses for the material. Most of the research has been conducted in Asian and European countries, but potential commercial uses for propolis have been acknowledged in the United States of America.

In a relatively brief article by Dr. F. B. Wells published in the November, 1976 issue of the American Bee Journal at pages 512, 513 and 542, the potential for use of propolis and propolis-containing preparations for therapeutic purposes is outlined. As is indicated in that article, and the foot notes to the article, the majority of research on propolis has been undertaken in Great Britain, Denmark, Poland, Russia, Romania, Czechoslovakia, East Germany, Yugoslavia and Bulgaria. These reports are in general agreement that raw propolis consists essentially of approximately 55% resins and balsams, 30% wax, 10% ethereal oils and 5% pollen. However, it will be appreciated that the composition of propolis may vary and is dependent upon the geographic area and season of its collection. Nevertheless, reported laboratory and clinical tests are quite consistent in their observations that propolis and propolis-containing compositions do exhibit bactericidal effects. It has been suggested that propolis may be responsible for the relatively low concentration of bacteria and moulds in the atmosphere within bee hives. As early as 1965 three Romanian investigators reported that alcoholic extracts of propolis, drones and royal jelly had viricidal activity against Type A influenza virus *in vitro*. Russian Patent No. 267014 claims the efficacy of an alcoholic extract of propolis in combination with glycerin for treating conjuctivitis. Russian Patent No. 232470 discloses and claims an alcoholic extract of propolis as part of a toothpaste composition possessing both prophylactic and antiseptic properties. Romanian Patent No. 48101 relates to a cosmetic lotion including an alcoholic extract of propolis also including boric acid. British Patent No. 1,465,194 teaches a method comprising repetitive freezing and thawing of propolis to obtain a material suitable for subsequent therapeutic uses.

Also forming part of the prior art is a paper by T. A. Schub, K. A. Kagramanowa, G. L. Malimon and G. Ja. Kiwman entitled "Antimicrobial action of Propaceum ointment as a function of the method of its preparation (Experience exchange)" published in Chim.-farm. Sh. (Moskau), 12 (11), S.95—96 (1978) and abstracted in Biological Abstracts, Vol. 68, 1979, Abstract No. 72464. In that paper the authors describe their experience and experiments relating to the antimicrobial effect of propolis extracts obtained by extracting raw propolis with various concentrations of ethyl alcohol, viz. concentrations of 40, 70 and 95%. Thick propolis extracts (TPE) were obtained, and in each case were tested for antimicrobial activity against *Bacillus cereus* 8035. They show that antimicrobial activity increases with extractant concentration, and recommend as the most suitable extractant 95% ethanol. They also conclude that the presence of water in the extractant is undesirable.

Thus, the preparation of alcoholic extracts containing propolis together with the use or organic solvents to prepare the extracts, is well known. Nevertheless, the literature fails to teach controlled reproducible methods for extracting propolis of known constituent composition. There is virtually no teaching in the prior art of any means for obtaining a water-soluble propolis extract, and the literature repeatedly refers to propolis as being substantially insoluble.

In one aspect, the present invention provides an improved method of extracting propolis as a dry powder from raw propolis or unprocessed beeswax which comprises the steps of:

(a) contacting the raw propolis or unprocessed beeswax with from 1 to 1.5 litres, per 500g of propolis or beeswax, of a non-aqueous organic solvent selected from ethyl alcohol, isopropyl alcohol, *n* - butyl alcohol, *sec* - butyl alcohol, *t* - butyl alcohol, ethyl ether, benzyl alcohol, propylene glycol, dimethyl sulfoxide, ethylene glycol, *n* - propyl alcohol, methyl alcohol, benzyl benzoate, acetone, polyethylene glycol or glacial acetic acid, and mixtures of two or more thereof;

(b) maintaining the resulting mixture at a temperature in the range 0°C to 37°C for from 1 to 10 days with periodic agitation to obtain a propolis-containing solution;

(c) filtering the solution to obtain a propolis-containing filtrate;

(d) cooling the propolis-containing filtrate to a temperature of at most −20°C;

(e) holding the filtrate at that temperature for about 24 hours with continuous agitation of the cooled filtrate;

(f) refiltering the cold filtrate whilst at that temperature, thereby to obtain a refiltered filtrate; and

(g) removing the solvent from the refiltered filtrate to obtain a purified propolis extract in the form of a dry, organic solvent soluble powder.

According to this aspect of the invention a dry purified propolis extract is obtained which is solvent soluble. This product is obtained by carrying out a double filtration process as follows: cooling the propolis-containing filtrate from the solvent extraction process to a temperature of at most −20°C; maintaining the cooled filtrate at said temperature for about 24 hours; agitating the cooled filtrate; re-filtering the cooled filtrate while maintaining the filtrate at that temperature and finally evaporating the re-filtered filtrate to dryness.

In a second aspect of the present invention, a dry, water-soluble propolis extract is obtained by the steps of:

(a) contacting the raw propolis or unprocessed beeswax with from 1 to 1.5 litres, per 500 g of propolis or beeswax, of a 10—25% aqueous solution of one or more of the following: ethyl alcohol, isopropyl alcohol, sec - butyl alcohol, tert - butyl alcohol, ethyl ether, propylene glycol, dimethyl sulphoxide, ethylene glycol, n - propyl alcohol, methyl alcohol, acetone, polyethylene glycol, and glacial acetic acid;

(b) maintaining the resulting mixture at a temperature in the range 0°C to 37°C for from 1 to 10 days with periodic agitation to obtain a propolis-containing solution;

(c) filtering the solution to obtain a propolis-containing filtrate; and

(d) removing the aqueous solvent from the filtrate, thereby to recover a propolis extract in the form of a dry, water-soluble powder.

By the methods of the present invention either a dry solvent soluble propolis powder can be obtained suitable for a variety of uses including, but not limited to, cosmetic and therapeutic application, or alternatively a water-soluble dry propolis powder can be obtained suitable for similar purposes. The particular solvent used in the extraction method will be chosen with regard to the intended end use of the dry propolis powder and whether or not it is desired that the powder be water-soluble or organic-solvent-soluble.

A practical procedure for preparing the dry propolis powder involves placing a quantity of raw material consisting essentially of either clean raw propolis or unprocessed beeswax in an amber glass container and covering it with solvent. If clean raw propolis is used as the raw material, 2 liters of solvent is added per kilogram or propolis. If unprocessed beeswax is used as the raw material, preferably 3 liters of solvent is used per kilogram of beeswax.

As will be set forth in greater detail hereinafter, preferred solvents for use in the method of this invention are absolute ethanol (ethyl alcohol) and aqueous solutions of ethanol. However, laboratory experimentation has shown that the following organic solvents, as well as aqueous solutions thereof (excluding benzyl benzoate by reason of its water insolubility) may also be used: isopropanol, n - butanol, sec - butanol, t - butanol, diethyl ether, benzyl alcohol, propylene glycol, dimethyl sulphoxide, ethylene glycol, n - propanol, methanol, benzyl benzoate, acetone, polyethylene glycol and glacial acetic acid.

It is of course to be understood that the solvent utilized should be of high quality and purity, consistent with the final uses of the dry propolis powder.

While the initial extraction preferably takes place at room temperature, acceptable results are given at temperatures from 0°C to 37°C. The extracting vessel containing the raw material and solvent is shaken several times daily for a period of from one to ten days, preferably at least three days. At the end of this time, the extract is filtered through Whatman No. 1 filter paper, or its equivalent. At this point it should be noted that the once-extracted raw material may be again covered with solvent and re-extracted as described above to obtain additional propolis-containing filtrates.

The solvent may then be removed from the propolis-containing filtrate to yield a dry propolis powder. Removal of the solvent may be accomplished by lyophilization or evaporation. Depending upon the solvent used in carrying out the extraction method, the resulting dry propolis powder will be either water-soluble or organic-solvent-soluble. For example, if ethanol is used as the solvent, an organic-solvent-soluble propolis powder will be obtained. However, if a 10%—25% aqueous ethanol is used as the solvent, water-soluble dry propolis powder will be obtained.

The purification of the propolis extract prior to obtaining the final dry propolis powder may be carried out by a preferred procedure which comprises first cooling the propolis-containing filtrate to a temperature of at most −20°C for approximately 24 hours. At the end of this period the extract will become viscous and opaque. While maintaining the filtrate at about −20°C it is shaken and filtered through Whatman No. 50 filter paper, or its equivalent. During the cold filtration procedure, which is carried out at about −20°C, a very clear filtrate should be obtained, and the waxy material remaining on the filter paper contains waste materials. If the filtrate is not clear, the cold filtration must be repeated.

The clear filtrate resulting from the cold filtration process may be filtered at the reduced temperature or may be brought to room temperature and filtered through a 0.2 micrometre filtration system. The solvent is then removed from this final filtrate, e.g. by lyophilization or evaporation, to obtain the purified dry propolis powder. In light of the relatively low temperatures utilized in the purification method outlined above, it is to be understood that this purification method is not suitable for aqueous solvent solutions, since the water would freeze.

While the above procedure has consistently proved to yield an extremely pure dry propolis powder of consistent composition, further quality control over the final powdered product may be obtained in the following fashion. After the cold filtration process described above, but prior to removing the solvent, the cold process filtrate may be brought to an extremely low temperature such as, for example, −70°C. If, after about 24 hours at that temperature, the filtrate is still clear, purity of the final product is assured. Any cloudiness in such a sample would indicate the unacceptability of the filtrate for further processing.

Detailed illustrative examples of various extraction and purification methods in accord with this invention are presented below. As will be set forth in greater detail, the resulting dry propolis powder may be utilized as a constituent in many cosmetic and therapeutic subtances. Accordingly, the present invention also extends to cosmetic, pharmaceutical or medicinal preparations comprising the propolis powder obtained by the method of this invention in admixture with a cosmetic or pharmaceutically acceptable topically administrable carrier, and orally administrable pharmaceutical and medicinal preparations comprising the powder in admixture with a non-toxic orally administrable carrier.

Example I

500 grams of clean raw propolis were placed in an amber glass container and covered with 1 liter of 15% aqueous ethanol solution. This mixture was allowed to stand for ten days at room temperature with periodic agitation several times each day. At the end of ten days, the mixture was filtered through Whatman No. 1 filter paper. The solvent was then removed from the propolis-containing filtrate by lyophilization (freeze drying) after partial reduction of the alcohol content by evaporation. This resulted in a dry propolis powder which was water soluble. Chemical analysis of the water soluble dry propolis powder gave the following results per 100 grams of dry propolis powder:

| | |
|---|---|
| Calcium | 0.33 grams |
| Phosphorous | 0.111 grams |
| Albumin | 3.7 grams |
| Protein | 18.5 grams |
| Creatinine | 118.5 milligrams |
| Bilirubin | 55.5 milligrams |
| Glucose | 26.1 grams |
| Alkaline phospatase | 4,148 International units |
| Potassium | 0.397 grams |
| Sodium | 0.085 grams |
| Zinc | 0.299 milligrams |
| Vitamin $B_{12}$ (estimation) | 0.133 milligrams |
| Folic acid (estimation) | 1.926 milligrams. |

Furthermore, study resulting from application of the resulting water soluble propolis to smooth muscle tissue revealed that this propolis contained no antihistamine properties quite unlike most drugs used today for treating virus symptoms. The presence of creatinine, bilirubin and alkaline phosphatase in the dry propolis powder is quite remarkable and may provide the basis for other uses of the powder, since these are normally found in animal tissue.

Example II

The method of Example I was repeated utilizing 10, 20 and 25% aqueous ethanol solutions as the solvents. The results in each case were substantially identical to those of Example I.

Example III

The method of Example I was repeated utilizing a 30% aqueous ethanol solution as the solvent. The propolis-containing filtrate derived according to this method was deemed unsuitable because of excessive cloudiness. It is therefore believed that water soluble dry propolis powder may only be obtained using aqueous solvent solutions of no more than about 25% concentration.

Example IV

500 grams of clean raw propolis were placed in an amber glass container and covered with 1 liter of absolute ethanol. The mixture was allowed to sit at room temperature for ten days with shaking several times each day. At the end of the ten days the extract was filtered through Whatman No. 1 filter paper.

To further purify the propolis-containing filtrate, it was cooled to a temperature of −20°C for 24 hours. At the end of this period it was observed that the extract had become viscous and opaque. The chilled filtrate was then shaken and filtered while maintaining the temperature at −20°C, through Whatman No. 50 filter paper. This resulted in a very clear purified propolis-containing filtrate. The solvent was removed from the purified filtrate by evaporation at 70°C for 48 hours until dry. The resulting material was organic solvent soluble dry propolis powder.

Equivalent results were obtained by repeating the above procedure with the following solvents:
   isopropyl alcohol
   sec-butyl alcohol
   tert-butyl alcohol
   ethyl ether
   benzyl alcohol
   propylene glycol
   dimethyl sulfoxide
   ethylene glycol
   n-propyl alcohol
   methyl alcohol
   benzyl benzoate
   acetone
   polyethylene glycol, and
   a mixture of equal parts ethyl alcohol and methyl alcohol.

Example V

Utilizing 100 grams unprocessed beeswax as the raw material and 300 ml ethanol as the solvent, the method of Example IV was repeated and yielded similar results. Of course, a smaller quantity of purified dry propolis powder was obtained.

Example VI

The method of Example IV was repeated; however, the solvent was removed from the purified filtrate by lyophilization.

Example VII

The following procedures were conducted in order to determine the bactericidal activity of the purified propolis powder obtained in accordance with the procedures set forth above in Example IV.

A 10%, by weight, dry propolis powder solution in absolute ethanol was prepared. This 10% solution was then used to prepare a variety of additional solutions, diluted with distilled water, to give final concentrations of dry propolis powder of from less than 10 milligrams to 10 milligrams of propolis powder per milliliter of solution. These solutions were then again diluted with a microbiological culture medium such as supplemented peptone broth II obtained from The Becton Dickinson Corporation of Rutherford, New Jersey, United States of America. The final solutions with peptone broth II varied from less than 1 to 10 milligrams of propolis per milliliter of peptone broth II solution. After about 24—48 hours incubation at about 37°C with each of the organisms listed below, the cultures were then replanted on the blood agar plates prior to a second incubation at about 37°C for about 24 hours. The three organisms utilized were present at a level of 15 million per 1 milliliter and were:

| | |
|---|---|
| Staphylococcus aureus | ATCC 25923 |
| Escherichia coli | ATCC 35933 |
| Pseudomonas aeruginosa | ATCC 27853 |

With the organisms tested individually in controlled studies, the propolis was found to have the following effects. Purified propolis powder in the final concentration of 2 milligrams per milliliter is lethal to Staphylococcus aureus. Purified propolis powder in a final concentration of 6 milligrams per milliliter is lethal to Escherichia coli. Purified propolis powder in a final concentration of 5 milligrams per milliliter is lethal to Pseudomonas aeruginosa.

Similar experimentation was performed utilizing water soluble propolis powder obtained in accordance with the method of Example II. The bactericidal activity of this propolis was equivalent to that just stated above.

Example VIII

The following procedures were followed in order to determine the antioxidative properties of water soluble dry propolis powder obtained in accordance with the procedures of Example II. 250 micrograms of the water soluble dry propolis powder were dissolved in 1 ml of distilled water. To this was added 0.02 ml of 0.1 N potassium permanganate. Decolorization took place in approximately 2.1 seconds giving a positive indication of the antioxidative properties of the water soluble dry propolis powder. These results are deemed important for the reason that the antioxidative properties of the propolis powder correlate to its bactericidal efficacy.

Dry propolis powder obtained in accordance with the method of this invention is concerned as a storage material available for immediate use. It is believed that the stability of propolis powder, when stored in an amber glass container at room temperature, is at least 10 years. The propolis powder is soluble in all the solvents mentioned in the methods given in the above examples including, obviously, water. Furthermore, organic propolis solutions are also soluble in glycerol and many chemical surfactants used in the pharmaceutical, cosmetic and food industries. Similarly, organic solutions of propolis powder are readily miscible with castor oil. The incorporation of organic solutions of propolis powder and different vegetable oils can be achieved first through the combination of the organic solution with castor oil or with chemical surfactants. Mineral oil, after combination with vegetable oil, creates suitable conditions for the addition of organic propolis solutions. Similar results can be achieved through the combination of organic propolis solutions with chemical surfactants and then with mineral oil.

Use of the dry propolis powder in organic or aqueous solutions, creams, lotions, suppositories, douches or in other pharmaceutical or cosmetic bases possess significant antibacterial, antifungal and antiviral activity. The observed antiviral activity of the dry propolis powder is particularly significant, and has been observed to be effective against Herpes simplex (type 1), Herpes simplex (type 2), Zoster virus, Epstein Barr virus and common cold viruses. Infectious hepatitis and distemper have not as yet been investigated, but theoretical considerations strongly indicate the efficacy of the extracted dry propolis powder.

In relatively large concentrations propolis extracted according to the method of this invention possesses anesthetic properties. Large concentrations also enhance animal tissue metabolism and may be applied locally to increase blood circulation. The increase in tissue metabolism during the treatment indicates that the stimulated tissue may react against inflammatory processes and that the propolis possesses regenerative properties. Increased blood circulation in local tissue in an important factor in combatting cellulites, cramps and other conditions. As will be indicated below, the oral application of 5 to 10% propolis powder in absolute ethanol has proved to be effective for bacterial, fungal and viral diseases.

Of particular note is the efficacy of such solutions in the treatment of Herpes simplex (types 1 and 2). Herpes simplex (type 1) is spread by contact of the mucous membranes which results in cold sores. Herpes simplex (type 2) infections are spread by sexual contact, and are considered as the most common veneral disease in the United States. It is generally accepted that Herpes simplex (type 2) is linked with cervical cancer. Both of the Herpes viruses can cause encephalitis which has a high mortality rate. Propolis powder prepared in accordance with the method of this invention and applied in the form of creams or ointments rapidly cures cold sores. Herpes simplex (type 2) infections of the sex organs can be cured by a specially applied douche. In both cases, subsequent oral treatment is recommended in order to prevent recurrence of the diseases.

*In vitro* studies of Herpes simplex (types 1 and 2) have demonstrated that about 10 micrograms of the propolis powder per milliliter of the culture media kills the viruses without affecting cell division. Oral administration in dosages of about 2 to 3 grams per day will prevent further outbreaks.

It should also be noted that the dry propolis powder obtained in accordance with the method of this invention has been observed to be extremely efficacious against the majority of common cold viruses when treated immediately after the first symptoms of the cold appear (no later than 2 or 3 hours after the onset of the condition). When the propolis is taken more than about 3 hours after symptoms appear, the severity of the symptoms do appear to diminish.

The following examples, then, are given for the purpose of illustrating various formulations for topical and oral administration of the dry propolis powder.

Example IX
Petrolatum propolis ointment
A 0.5 to 5.0% propolis-ethanol solution may be incorporated into white or yellow petrolatum in concentrations up to 60%, by volume, propolis solution. To decrease the viscosity of this and other ointments, mineral oil, wool fats, animal fats or fish fats can be used.

Example X
Spermaceti cream
The following ingredients are mixed to obtain the cream, with all composition constituents listed in weight percents:

| | |
|---|---|
| Polyoxyethylene 20 sorbitan monostearate | 8.0% |
| Propolis powder | 2.0% |
| Sorbitan monostearate | 8.0% |
| Spermaceti | 10.0% |
| Water q.s. ad | 100.0% |

Example XI
Anti-herpes cream I
The following formulation, with constituents listed in weight percents, has proved to be effective in treating Herpes viruses:

| | |
|---|---|
| Propolis powder | 1.0—5.0% |
| Dermabase q.s. ad | 100.0% |

Dermabase (trademark) is a hypo-allergenic cream base which is compatible with most medicaments. Its pH is close to that of human skin. Dermabase is a vehicle for topical application used in pharmaceutical, cosmetic and veterinary preparations. It is manufactured by Professions Pharmaceutical Corporation, 2795 Bates Road, Montreal, Quebec H3S 1B6, Canada.

Example XII
Anti-herpes cream 2
The following formulation with constituents listed in weight percents, has proved to be effective in treating Herpes viruses:

| | |
|---|---|
| Propolis powder | 0.5—10.0% |
| Unibase q.s. ad | 100.0% |

Unibase (trademark) is a dermatological all-purpose base, and has a pH approximating that of the skin. Unibase is manufactured by Parke-Davis & Company, Limited, Box 633, Station "A", Scarborough, Ontario M1K 5C5, Canada.

Example XIII
Suppositories
The propolis powder prepared in accordance with the method of this invention may be administered in suppositories prepared in accordance with the following formula wherein all constituents are listed in weight percents:

| | |
|---|---|
| Polyoxyethylene 2-sorbitan monostearate | 35.0—55.0% |
| Polyoxyethylene 4 sorbitan monostearate | 40.0—60.0% |
| Propolis powder | 1.0—5.0% |

Example XIV
Suppositories
Yet another formulation for suppositories including powdered propolis may be prepared as follows, again with all constituents listed in weight percents:

| | |
|---|---|
| Glyceryl laurate | 6.0—16.0% |
| Polyoxyethylene 4 sorbitan monostearate | 82.0—92.0% |
| Propolis powder | 2.0% |

Example XV
Oral preparations
A liquid (syrup) preparation for the oral administration of propolis powder may be prepared according to the following formula:

| | |
|---|---|
| Polyoxyethylene 20 sorbitan monooleate | 60 grams |
| 50% Propolis ethanol solution | 30 grams |
| Propylene glycol | 100 grams |
| Ethyl alcohol | 100 grams |
| 70% aqueous sorbitol solution, USP* | 300 grams |
| Distilled water | 410 grams |

*USP=United States Pharmacopoeia.

Flavouring agents and/or preservatives may be included as desired.

The following Examples are presented for the purpose of setting forth propolis-containing solutions which have proved to be efficacious for treating the common cold.

Example XVI

10 milliliters (approximately 1 tablespoon) of about 10% propolis-ethanol solution are mixed in a glass (8 ounces:237 ml) of water or juice. Alternatively, the propolis-ethanol solution may be mixed with coffee or tea giving the appearance of added milk.

This mixture may be taken about 3 times a day, but if first administered immediately after the first symptoms are noted, the symptoms may disappear within a few hours.

Example XVII
Nasal ointment for cold or hay fever

| | |
|---|---|
| Petrolatum | 96.0 grams |
| 50% Propolis-ethanol solution | 4.0 grams |

This ointment should be applied to the nostrils a few times daily as required. Other similar bases may be substituted for the petrolatum.

Example XVIII
Honey-propolis formula

| | |
|---|---|
| Honey | 80.0—90.0 grams |
| 25% Propolis in concentrated propylene glycol, USP | 10.0—20.0 grams |

Example XIX
Gelatin capsules

| | |
|---|---|
| Sorbitol solution, USP | 80.0—90.0 grams |
| 25% Propolis in concentrated propylene glycol, USP | 10.0—20.0 grams |

The above is only one example of many possible means of incorporating propolis into capsules. The propylene glycol USP potentiates the activity of alcoholic propolis solutions up to 6.5 fold with respect to cidal activities versus numerous microorganisms.

It has also been determined that propolis obtained in accordance with the methods of this invention is useful in the treatment of respiratory tract infections and inflammatory processes of the lungs, including bronchial asthma, sinusitis and hay fever. One means of treatment is inhalation therapy in which the drug is dissolved in hot water, and the vapors are inhaled according to conventional procedures. The following example sets forth a formula and procedure for the inhalation of propolis.

Example XX
About 10 milliliters (approximately 1 tablespoon) of 10% alcoholic solution of propolis is added to about 1 liter of hot water and mixed well. The vapors are inhaled.

Example XXI
Yet another formulation for inhalation therapy may be prepared as follows:

| | |
|---|---|
| 50% propolis-ethanol solution | 10 grams |
| Gum benzoin | 8 grams |
| Storax | 6 grams |
| Tolu balsam | 2 grams |
| Aloe | 2 grams |

The above is blended into 100 milliliters of 90% ethanol. In a preferred use, 1 teaspoon of this mixture is added to 500 milliliters of hot water to produce the inhalation solution.

Other solvents of propolis for inhalation therapy include benzyl alcohol and polyethylene glycol.

In studies of asthma, in experimental animals it has been determined that polyethylene glycol may be used as a solvent for propolis in the formulation of injectable preparations (intramuscular).

As indicated above in Example XIX, propolis in gelatin capsules with suitable non-ionic surfactants such as, for example, polyoxyethylene 20 sorbitan monostearate, can be used in the treatment of gastrointestinal tract infections, ulcers and other inflammatory disorders of the bowel. Treatment can be achieved with the oral application of propolis in conjunction with non-ionic surfactants and propylene glycol in the form of gelatin capsules. Yet another treatment form comprises the use of retention enemas including propolis.

The gelatin capsules should be formulated to maximize the absorption of propolis in the gastrointestinal tract. This can be achieved by mixing the propolis-ethanol solution with sorbitol or diluted propylene glycol solution until a milky appearance of the combined substances results. This will avoid or minimize coating of the gastric mucosa.

The daily intake of propolis should range within about 3—6 grams. The retention enemas should be given in quantities of about 200 milliliters three times a week or according to a physician's directions. The pH of the enema solution should be between 5.0 and 6.0. The concentration of propolis may vary from about 5 to about 10 percent.

It has also been determined that the propolis can be mixed with concentrated propylene glycol USP and incorporated in the gelatin capsules which, when ingested, can coat the stomach walls. The capsules in contact with gastric juice will form a milky suspension which absorbs over the surface of the stomach cells forming a thin film. This coating has been shown to suppress the appetite for 2 to 3 hours, and such treatment is recommended for the control of weight in obese patients.

Vaginitis, cervicitis and cervical erosions may be treated with 1 to 5% propolis-ethanol solution in combination with glycerine, propylene glycol and with or without non-ionic surfactants in the form of douches, suppositories or ointments.

Propolis powder can be used in otolaryngology and renal infections in the form of solutions, tablets or capsules.

Propolis powder can be incorporated into ointments for the treatment of burns.

For cystic fibrosis the daily oral application of one gram of propolis powder should be administered in the form of capsules or solutions.

Patients with leukocyte dysfunction disorders often develop recurrent bacterial infections which cannot be controlled by current methods of therapy. Propolis powder solutions, being strong bactericidal agents, can be used orally in the treatment of these disorders. The oral application of propolis powder solutions in a daily dose of 2 to 3 grams calculated on an anhydrous basis for a period of ten days is recommended. They can also be used in conjunction with current antibiotic therapy.

The regenerative and rejuvenating properties of propolis powder can be used in areas of medical science such as plastic surgery and dental surgery. Propolis powder in the form of ointments, creams, lotions, solutions, shampoos, cream rinses, shaving lotions and scalp creams in contact with the damaged or diseased lesion on the animal or human body shows considerable healing and rejuvenating properties. These regenerative properties of propolis powder can be observed in dental surgery, plastic surgery of animal organs and also *in vitro* tissue culture studies.

When propolis powder solutions are added to a fibroblast cell culture, the number of mitotic cells increases. Such studies demonstrate an increase in the enzymes responsible for the increased metabolism within the cell. The increased enzyme activity occurs with the following enzymes:

Andenosine triphosphatase
Acid phosphatase
Glucose-6-phosphatase
Succinate dehydrogenase This may explain why propolis powder solutions show regenerative properties such as the increase in the activity of formulation of a collagen.

Propolis powder may be consistently employed in conjunction with other antibacterial agents used in densistry. Propolis powder incorporated in suitable bases will control superficial and deep infections of the mucous membranes and bone, and will disinfect tooth cavities or root canals.

Propolis powder solutions used alone or in combination with benzocaine is an excellent anaesthetic. An illustrative formula follows.

Example XXII
Propolis benzocaine solution

Ethyl aminobenzoate            7.5 grams

Propolis powder in propylene glycol to make 150 milliliters.

The final concentration of propolis in the above formula may vary from 1.0 to 10.0%, by weight.

Propolis powder may be used in the form of liquides or pastes to relieve post extraction pain (alveolar analgesic). An example of a paste follows.

Example XXIII
Propolis benzocaine paste

| Lanolin alcohols | 10.0 grams |
|---|---|
| Yellow beeswax | 10.0 grams |
| Petrolatum | 10.0 grams |
| Ethyl aminobenzoate | 2.0 grams |
| Clove oil | 3.0 grams |
| 50% Propolis ethanol solution | 15.0 grams |

Propolis in the above formula acts as antimicrobial, analgesic, anaesthetic and regenerative agents. It speeds regeneration of the tissues and reduces inflammatory infiltrations.

Example XXIV
Propolis in a mouth rinse solution

| 2—10% Propolis in alcohol-glycerol solution | 40.0%, by volume |
|---|---|
| Propylene glycol | 10.0%, by volume |
| Distilled water | 49.8%, by volume |
| Flavouring agent | 0.2%, by volume |

In this formula sorbitol USP or non-ionic surfactants may be used. Propolis powder has wide ranging applications in the treatment of dermatological disorders. Propolis powder for this purpose can be incorporated into ointments, creams, lotions, solutions, shampoos, cream rinses, douche and oral preparations. Propolis powder can also be incorporated into currently used pharmaceutical and cosmetic products directly or with suitable surfactants.

Ringworm may be treated by the local application of 10% propolis-alcohol solution or 5 to 10% propolis powder in an ointment made of petrolatum or another base two to three times daily for four weeks, or until symptoms are no longer evident.

Psoriasis, seborrheic dermatitis, eszema and neurodermatitis may be successfully treated with from 0.5 to 25% propolis powder in the form of lotions, solutions or ointments prepared with bases as previously described.

Propolis powder in concentrations from 0.5 to 10.0% may be formulated with wool fat or its alcohols and in combination with petrolatum and mineral oil for treating corns, warts and calluses.

Propolis concentrations of from 0.25 to 1.0% combined with non-ionic surfactants and incorporated into shampoos, cream rinses or creams are efficacious for treating dandruff. Similarly, propolis solutions in concentrations varying from 0.25 to 2.0% may be incorporated into hypoallergenic bases for treating poison ivy and jellyfish dermatitis.

Acne vulgaris may be treated with up to 3% propolis solutions combined with up to 3% salicyclic acid, non-ionic surfactants such as polyoxyethylene 20 sorbitan monolaurate and with 40% ethyl alcohol or another suitable alcohol. Other compounds can also be used such as methyl salicylate, glyucerine, or propylene glycol.

A most effective treatment for hermatomas and other bruises consists of about 2—5% propolis-ethanol solution made into an ointment with petrolatum. The ointment is spread on the bandage, which is then taped over the affected area.

The unique properties of propolis have been used to create a new range of cosmetics. Propolis powders and solutions are endowed with preservative and antioxidant properties and natural skin rejuvenating properties which are indispensible to the many cosmetic preparations. The concentration of propolis in its powdered form can vary from less than 1% to greater than 2%. As a preservative or an antibacterial and antifungal agent propolis solutions may be used in concentrations varying from 1.0 to 2.0% on an anhydrous basis. In antiwrinkle preparations the percentage of propolis powder can be increased. The rejuvenating and other properties of propolis in trials of a number of cosmetic formulas have fulfilled expectations.

Propolis powders may be used in moisturizers, night and day creams, nutrient creams, barrier creams, cuticle creams, cleansing creams, lotions, cold creams, mask preparations, all types of lotions and solutions, shampoos, conditioners, cream rinses, shaving lotions, finger nail polish, soaps, lipsticks, baby creams, baby lotions, anti-diaper rash products, massage creams, massage lotions, skin rubbing products, aerosols for cosmetic or medical purposes, keratolytic (desquamating) products and anti-cellulite products.

Moisturizing creams are used during the day under make-up or as a foundation cream endowing the skin with a soft appearance, retaining its moisture and preventing wrinkles. An acceptable formulation for such a moisturizing cream follows:

Example XXV

| | |
|---|---|
| Cream base | 78.0 grams |
| Propylene glycol | 10.0 grams |
| Avocado oil | 10.0 grams |
| 50% Propolis solution | 2.0 grams |

Propylene glycol may be substituted with glycerol, sorbitol solution USP or lanolin. Avocado oil may be substituted with another vegetable oil, or an animal or fish fat.

Example XXVI
Cold creams
These contain large amounts of fatty or oil ingredients. An example follows:

| | |
|---|---|
| Cream base | 40.0 grams |
| Oil or fat | 58.0 grams |
| 50% Propolis solution | 2.0 grams |

Example XXVII
Night cream

| | |
|---|---|
| Cream base | 70.0 grams |
| Oil or fat | 27.0 grams |
| 50% Propolis solution | 3.0 grams |

The increased quantity of propolis is an important factor in the rejuvenation of the skin during the night when the mitotic activity of the skin is increased. Humectants may be added to aid in retaining skin moisture.

Example XXVIII
Cuticle cream
This softens the cuticles and prevents the nails from becoming brittle. It actually toughens and thickens the nails permitting them to be grown longer. A formula follows:

| | |
|---|---|
| Petrolatum-lanolin base | 97.0 grams |
| 50% Propolis solution | 3.0 grams |

Example XXIX
Facial masks
Facial masks are intended as skin cleansing and tightening agents. The presence of propolis aids in rejuvenating the skin. The following is one example of a facial mask:

| | |
|---|---|
| Fuller's earth | 50.0%, by volume |
| 50% Glycerol solution | 44.0%, by volume |
| 50% Propolis solution | 5.7%, by volume |
| Perfume, if desired | 0.3%, by volume |

China clay, kaolin or bentonite may also be used in combinations with or as a replacement of the Fuller's earth used above.

Propolis powder may also be used in liniments in conjunction with oil of turpentine, capsicum, extracts or arnica, linseed oil, camphor and isopropyl alcohol, etc.

As previously indicated, propolis powder and propolis solutions may be used as preservatives, anti-oxidants, stabilizers and rejuvenating agents in a variety of current cosmetic products. Their incorporation would require only slight modifications of the original formulas. The propolis may be directly incorporated or via the use of a solvent or surfactant. The surfactants can be non-ionic, anionic or cationic.

Propolis powder and solutions may also be used in the food industry as preservatives, anti-oxidants and stabilizers of food products, with emphasis on preserving animal and fish fats. In the distilling industries they can be incorporated into alcohol for use in oral treatment of patients. In the tobacco industry it is believed that can be used as a flavour or as a medicated agent in antiasthmatic cigarettes.

Briefly summarizing, then, it can be seen that the present invention presents a unique method for preparing dry propolis powders which are suitable for a wide variety of end use applications. It is to be emphasized that the method and resulting water soluble propolis powder is deemed quite significant for the reason that heretofore water soluble forms of propolis were not known.

**Claims**

1. A method of obtaining a propolis extract by solvent extraction from raw propolis or unprocessed beeswax followed by filtration of the solvent extract, and evaporation of the solvent from the filtrate, characterised by the steps of:

(a) contacting the raw propolis or unprocessed beeswax with from 1 to 1.5 litres, per 500 g of propolis or beeswax, of a non-aqueous organic solvent selected from ethyl alcohol, isopropyl alcohol, $n$ - butyl alcohol, $sec$ - butyl alcohol, $t$ - butyl alcohol, ethyl ether, benzyl alcohol, propylene glycol, dimethyl sulfoxide, ethylene glycol, $n$ - propyl alcohol, methyl alcohol, benzyl benzoate, acetone, polyethylene glycol or glacial acetic acid, and mixtures of two or more thereof;

(b) maintaining the resulting mixture at a temperature in the range 0°C to 37°C for from 1 to 10 days with periodic agitation to obtain a propolis-containing solution;

(c) filtering the solution to obtain a propolis-containing filtrate;

(d) cooling the propolis-containing filtrate to a temperature of at most −20°C;

(e) holding the filtrate at that temperature for about 24 hours with continuous agitation of the cooled filtrate;

(f) refiltering the cold filtrate whilst at that temperature, thereby to obtain a refiltered filtrate; and

(g) removing the solvent from the refiltered filtrate to obtain a purified propolis extract in the form of a dry, organic solvent soluble powder.

2. A method according to claim 1, characterised in that in step (a) the solvent used is absolute ethanol.

3. A method according to claim 1 or 2, characterised in that, in step (g), the solvent or solvent mixture is removed by evaporation at 55° to 70°C.

4. A method as claimed in claim 3 in which the evaporation takes place at 70°C.

5. A method as claimed in claim 4, characterised in that the purity of the cooled propolis-containing filtrate is verified before removal of the solvent in step (g) by further cooling the refiltered filtrate to a temperature of at most −70°C and maintaining it at that temperature for a period of about 24 hours; and observing the clarity of the purified propolis-containing filtrate, substantial clarity after the said period indicating purity of the final product.

6. A method of obtaining a propolis extract by solvent extraction from raw propolis or unprocessed beeswax using an aqueous-organic solvent mixture as the extraction medium, filtering the solvent extract, and evaporating the solvent from the filtrate, characterised by the steps of:

(a) contacting the raw propolis or unprocessed beeswax with from 1 to 1.5 litres, per 500 g of propolis or beeswax, of a 10—25% aqueous solution of one or more of the following: ethyl alcohol, isopropyl alcohol, $sec$ - butyl alcohol, $tert$ - butyl alcohol, ethyl ether, propylene glycol, dimethyl sulphoxide, ethylene glycol, $n$ - propyl alcohol, methyl alcohol, acetone, polyethylene glycol, and glacial acetic acid;

(b) maintaining the resulting mixture at a temperature in the range 0°C to 37°C for from 1 to 10 days with periodic agitation to obtain a propolis-containing solution;

(c) filtering the solution to obtain a propolis-containing filtrate; and

(d) removing the aqueous solvent from the filtrate, thereby to recover a propolis extract in the form of a dry, water-soluble powder.

7. A method as claimed in claim 6, in which the solvent is a 10—25% aqueous solution of ethyl alcohol.

8. A method as claimed in claim 7, in which the solvent is a 15% aqueous solution of ethyl alcohol.

9. A method according to claim 6, 7 or 8, wherein, in step (d), the aqueous solvent is removed by lyophilisation.

10. A cosmetic or topically administrable pharmaceutical or medicinal preparation comprising a propolis extract prepared by a method claimed in any one of claims 1—9 in admixture with a topically administrable cosmetic or pharmaceutically acceptable carrier.

11. An orally administrable pharmaceutical or medicinal preparation comprising a propolis extract prepared by a method claimed in any one of claims 1—9 in admixture with an orally administrable, non-toxic carrier.

**Patentansprüche**

1. Verfahren zur Gewinnung eines Propolisauszuges durch Lösungsmittelextraktion von roher Propolis oder unbehandeltem Bienenwachs, gefolgt von einer Filtration des Lösungsmittelauszuges, und Verdampfung des Lösungsmittels aus dem Filtrat, gekennzeichnet durch folgende Schritte:

(a) Inkontaktbringen der rohen Propolis oder des unbehandelten Bienenwachses mit 1 bis 1,5 Liter, pro 500 g Propolis oder Bienenwachs, eines nicht wäßrigen organischen Lösungsmittels ausgewählt aus Ethylalkohol, Isopropylalkohol, $n$ - Butylalkohol, $sec$ - Butylalkohol, $t$ - Butylalkohol, Ethylether, Benzylalkohol, Propylenglycol, Dimethylsulfoxid, Ethylenglycol, $n$ - Propylalkohol, Methylalkohol, Benzylbenzoat, Aceton, Polyethylenglycol oder Eisessig, und Mischungen aus zwei oder mehreren von diesen;

(b) Halten der resultierenden Mischung bei einer Temperatur im Bereich zwischen 0°C bis 37°C für ein bis zehn Tage unter periodischem Bewegen, um eine Propolis enthalende Lösung zu erhalten;

(c) Filtrieren der Lösung um ein Propolis enthaltendes Filtrat zu erhalten;

(d) Kühlen des Propolis enthaltenden Filtrates auf eine Temperatur von höchstens −20°C;

(e) Halten des Filtrates bei dieser Temperatur für etwa 24 Stunden unter kontinuierlicher Bewegung des gekühlten Filtrates;

(f) erneutes Filtrieren des kalten Filtrates, während es auf dieser Temperatur ist, und dadurch Erhalten eines nochmals filtriertem Filtrates; und

(g) Entfernen des Lösungsmittels von dem nochmals filtrierten Filtrat um ein gereinigtes Propolisextrakt in Form eines trockenen in organischem Lösungsmittel löslichen Pulvers zu erhalten.

2. Verfahren nach Anspruch 1, dadurch gekennzeichnet, daß das verwendete Lösungsmittel absolutes Ethanol ist.

3. Verfahren nach Anspruch 1 oder 2, dadurch gekennzeichnet, daß in Schritt (g) das Lösungsmittel oder die Lösungsmittelmischung durch Verdampfen bei 75° bis 70°C entfernt wird.

4. Verfahren nach Anspruch 3, wobei die Verdampfung bei 70°C stattfindet.

5. Verfahren nach Anspruch 4, dadurch gekennzeichnet, daß die Reinheit des gekühlten, Propolis enthaltenden Filtrates vor dem Entfernen des Lösungsmittels in Schritt (g) durch weiteres Abkühlen des nochmals filtrierten Filtrates auf eine Temperatur von höchstens −70°C und Halten bei dieser Temperatur für einen Zeitraum von etwa 24 Stunden und Beobachten der Klarheit des gereinigten Propolis enthaltenden Filtrates überprüft wird, wobei im wesentliche Klarheit nach dem genannten Zeitraum die Reinheit des Endproduktes anzeigt.

6. Verfahren zur Gewinnung eines Propolisauszuges durch Lösungsmittelextraktion aus roher Propolis oder unbehandeltem Bienenwachs unter Verwendung eines wäßrigen organischen Lösungsmittels als Extraktionsmittel, Filtern des Lösungsmittelauszuges und Verdampfen des Lösungsmittels aus dem Filtrat, gekennzeichnet durch folgende Schritte:

(a) Inkontaktbringen der rohen Propolis oder des unbehandelten Bienenwachses mit 1 bis 1,5 Liter, pro 500 g Propolis oder Bienenwachs, einer 10 bis 25%-igen wäßrigen Lösung von einem oder mehreren derfolgenden: Ethylalkohol, Isopropylalkohol, *sec* - Butylalkohol, *tert* - Butylalkohol, Ethylether, Propylenglycol, Dimethylsulfoxid, Ethylenglycol, *n* - Propylalkohol, Methylalkohol, Aceton, Polyethylenglycol und Eisessig;

(b) Halten der resultierenden Mischung bei einer Temperatur im Bereich 0°C für 1 bis 10 Tage unter periodischem Bewegen, um eine Propolis enthaltende Lösung zu erhalten;

(c) Filtrieren der Lösung, um ein Propolis enthaltendes Filtrat zu erhalten; und

(d) Entfernen des wäßrigen Lösungsmittels aus dem Filtrat, um dadurch einen Propolisextrakt in Form eines trockenen, wasserlöslichen Pulvers zu erhalten.

7. Verfahren nach Anspruch 6, bei welchem das Lösungsmittel eine 10 bis 25%-ige wäßrige Lösung von Ethylalkohol ist.

8. Verfahren nach Anspruch 7, wobei das Lösungsmittel eine 15%-ige wäßrige Lösung von Ethylalkohol ist.

9. Verfahren nach Anspruch 6, 7 oder 8, wobei unter Schritt (d) das wäßrige Lösungsmittel durch Gefriertrocknung entfernt wird.

10. Kosmetisches oder äußerlich anwendbares pharmazeutisches oder medizinisches Präparat, welches einen Propolisauszug aufweist, der nach einem Verfahren gemäß einem der Ansprüche 1 bis 9 unter Beimischung einer äußerlich anwendbaren kosmetisch oder pharmazeutisch verträglichen Trägersubstanz hergestellt ist.

11. Oral anwendbares pharmazeutisches oder medizinisches Präparat, welches einen Propolisauszug aufweist, der nach einem in einem der Ansprüche 1 bis 9 beanspruchten Verfahren unter Beimischung einer oral verabreichbaren, nicht toxischen Trägersubstanz hergestellt ist.

**Revendications**

1. Procédé d'obtention d'un extrait de propolis, par extraction aux solvants à partir de propolis brute ou de cire d'abeille non-traitée, suivie d'une filtration du produit de l'extraction aux solvants, et évaporation du solvant se trouvant dans le filtrat, caractérisé en ce qu'il comprend les étapes consistant

(a) à mettre en contact la propolis brute ou la cire d'abeille non-traitée avec 1 à 1,5 litres, par 500 g de propolis ou de cire d'abeille, d'un solvant organique non-aqueux choisi parmi l'alcool éthylique, l'alcool isopropylique, l'alcool n - butylique, l'alcool sec - butylique, l'alcool tert - butylique, l'éther éthylique, l'alcool benzylique, le propylèneglycol, le diméthylsulfoxyde, l'éthylèneglycol, l'alcool n - propylique, l'alcool méthylique, le benzoate de benzyle, l'acétone, le polyéthylèneglycol ou l'acide acétique glacial, et des mélanges de deux ou plus d'entre eux;

(b) à maintenir le mélange obtenu à une température dans l'intervalle de 0 à 37°C, pendant 1 à 10 jours, avec agitation périodique, pour obtenir une solution contenant de la propolis;

(c) à filtrer la solution pour obtenir un filtrat contenant de la propolis;

(d) à refroidir le filtrat contenant de la propolis, à une température au plus égale à −20°C;

(e) à maintenir le filtrat à cette température pendant environ 24 heures, en agitant en continu le filtrat refroidi;

(f) à refiltrer le filtrat froid, toujours à cette même température, de façon à obtenir un filtrat refiltré; et

(g) à chasser le solvant du filtrat refiltré pour obtenir un extrait de propolis purifié, sous la forme d'une poudre sèche, soluble dans les solvants organiques.

2. Procédé selon la revendication 1, caractérisé en ce que, dans l'étape (a), le solvant utilisé est l'éthanol absolu.

3. Procédé selon la revendication 1 ou 2, caractérisé en ce que, dans l'étape (g), le solvant ou le mélange de solvant est chassé par évaporation à une température de 55 à 70°C.

4. Procédé selon la revendication 3, dans lequel l'évaporation a lieu à 70°C.

5. Procédé selon la revendication 4, caractérisé en ce que la pureté du filtrat contenant de la propolis et refroidi est vérifiée avant élimination du solvant dans l'étape (g), par un nouveau refroidissement du filtrat refiltré, à une température au plus égale à −70°C, et maintien de ce filtrat à cette température pendant environ 24 heures; et observation de la clarté du filtrat contenant de la propolis et purifié, une clarté parfaite, au bout de cette période, indiquant la pureté du produit fini.

6. Procédé d'obtention d'un extrait de propolis par extraction aux solvants à partir de propolis brute ou de cire d'abeille non-traitée, consistant à utiliser comme milieu d'extraction un mélange de solvants organiques - aqueux, à filtrer le produit de l'extraction aux solvants et à chasser par évaporation le solvant du filtrate, caractérisé en ce qu'il comprend les étapes consistant:

(a) à mettre en contact la propolis brute ou la cire d'abeille non-traitée avec 1 à 1,5 litres, par 500 g de propolis ou de cire d'abeille, d'une solution aqueuse à 10—25% d'un ou plusieurs des solvants suivants: alcool éthylique, alcool isopropylique, alcool sec - butylique, alcool tert - butylique, éther éthylique, propylèneglycol, diméthylsulfoxyde, éthylèneglycol, alcool n - propyli-

que, alcool méthylique, acétone, polyéthylèneglycol et acide acétique glacial;

(b) à maintenir le mélange obtenu à une température comprise entre 0 et 37°C pendant 1 à 10 jours, en agitant périodiquement pour obtenir une solution contenant de la propolis;

(c) à filtrer la solution pour obtenir un filtrat contenant de la propolis; et

(d) à chasser le solvant aqueux du filtrat, en récupérant un extrait de propolis sous la forme d'une poudre sèche soluble dans l'eau.

7. Procédé selon la revendication 6, dans lequel le solvant est une solution aqueuse à 10—25% d'alcool éthylique.

8. Procédé selon la revendication 7, dans lequel le solvant est une solution aqueuse à 15% d'alcool éthylique.

9. Procédé selon la revendication 6, 7 ou 8, dans lequel, dans l'étape (d), le solvant aqueux est chassé par lyophilisation.

10. Préparation cosmétique, ou pharmaceutique ou médicale administrable par voie topique, comprenant un extrait de propolis préparé par un procédé selon l'une quelconque des revendications 1 à 9, en mélange avec un excipient pour cosmétique, ou pharmaceutiquement acceptable, administrable par voie topique.

11. Préparation pharmaceutique ou médicale, administrable par voie orale, comprenant un extrait de propolis préparé par un procédé selon l'une quelconque des revendications 1 à 9, en mélange avec un excipient non-toxique administrable par voie orale.